# FASCICULE DE BREVET EUROPEEN

(11) **EP 3 180 417 B1**
(45) Date de publication et mention de la délivrance du brevet: **02.10.2019**
(21) Numéro de dépôt: 15759885.5
(22) Date de dépôt: 04.08.2015
(51) Int. Cl.: C12M 1/34, C12M 1/42, C12Q 1/00, C12N 5/071, G01N 15/14

(54) **DISPOSTIF ET PROCEDE DE SELECTION DE CELLULES EUCARYOTES DANS UN CANAL DE TRANSPORT PAR ALTERATION DES CELLULES EUCARYOTES AU MOYEN D'UN RAYONNEMEMENT ELECTROMAGNETIQUE**
VORRICHTUNG UND VERFAHREN ZUR AUSWAHL EUKARYOTISCHER ZELLEN IN EINEM TRANSPORTKANAL DURCH VERÄNDERUNG DER EUKARYOTISCHEN ZELLEN MITTELS ELEKTROMAGNETISCHER STRAHLUNG
DEVICE AND METHOD FOR SELECTING EUKARYOTIC CELLS IN A TRANSPORTATION CHANNEL BY ALTERING THE EUKARYOTIC CELLS BY MEANS OF ELECTROMAGNETIC RADIATION

(30) Priorité: 11.08.2014 FR 1457734
(43) Date de publication de la demande: 21.06.2017
(73) Titulaire: Genes Diffusion, 59500 Douai (FR)
(72) Inventeur: LIEGEOIS, Luc, F-59283 Raimbeaucourt (FR); COURTADE, Emmanuel, F-59260 Lille-Hellemmes (FR); DAMART, Hervé, F-59118 Wambrechies (FR); PESEZ, Jean, F-59130 Lambersart (FR); THOMMEN, Quentin, F-59260 Lille-Hellemmes (FR); TREIZEBRE, Anthony, F-59310 Coutiches (FR)
(74) Mandataire: Matkowska, Franck
(86) Numéro de dépôt international: PCT/FR2015/052154
(87) Numéro de publication internationale: WO 2016/024061

(56) Documents cités:
- WO-A1-2005/075629
- WO-A1-2007/022641
- WO-A1-2009/151624
- WO-A1-2010/043917
- WO-A1-2010/099118
- WO-A2-2004/088283
- WO-A2-2010/001254
- JOHNSON L A ET AL: "THE BELTSVILLE SPERM SEXING TECHNOLOGY: HIGH-SPEED SPERM SORTING GIVES IMPROVED SPERM OUTPUT FOR IN VITRO FERTILIZATION AND AI", JOURNAL OF ANIMAL SCIENCE, AMERICAN SOCIETY OF ANIMAL SCIENCE, US, vol. 77, no. 2, 1 janvier 1999 (1999-01-01), pages 213-220, XP001025554, ISSN: 0021-8812
- KOH-ICHI HAMANO: "Sex Preselection in Bovine by Separation of X- and Y-Chromosome Bearing Spermatozoa", JOURNAL OF REPRODUCTION AND DEVELOPMENT, vol. 53, no. 1, 1 février 2007 (2007-02-01), pages 27-38, XP055163371, ISSN: 0916-8818, DOI: 10.1262/jrd.18141
- HAZOUT A ET AL: "Causes et implications cliniques des alterations de l'ADN des spermatozoides", GYNECOLOGIE OBSTETRIQUE & FERTILITE, ELSEVIER MASSON, FR, vol. 36, no. 11, 1 novembre 2008 (2008-11-01), pages 1109-1117, XP025675015, ISSN: 1297-9589, DOI: 10.1016/J.GYOBFE.2008.07.017 [extrait le 2008-10-28]
- ZAN-BAR T ET AL: "Influence of visible light and ultraviolet irradiation on motility and fertility of mammalian and fish sperm", PHOTOMEDICINE AND LASER SURGERY, MARY ANN LIEBERT, NEW ROCHELLE, NY, US, vol. 23, no. 6, 1 décembre 2005 (2005-12-01), pages 549-555, XP009103668, ISSN: 1549-5418, DOI: 10.1089/PHO.2005.23.549
- SILVA P F N ET AL: "Detection of damage in mammalian sperm cells", THERIOGENOLOGY, LOS ALTOS, CA, US, vol. 65, no. 5, 15 mars 2006 (2006-03-15), pages 958-978, XP027930357, ISSN: 0093-691X [extrait le 2006-03-15]

## Description

### Domaine technique

La présente invention concerne le domaine de la sélection in vitro de cellules eucaryotes, et en particulier, mais on exclusivement de spermatozoïdes, notamment de spermatozoïdes d'animaux, au moyen d'un rayonnement électromagnétique.

### Art antérieur

Un spermatozoïde est une cellule eucaryote de type haploïde, qui ne contient généralement qu'un seul exemplaire de chaque chromosome, par exemple un chromosome X ou Y, et dont la motilité est assurée par un flagelle.

Dans le domaine de la reproduction animale, on a développé à ce jour différentes solutions de sexage, qui permettent de sélectionner et trier automatiquement des spermatozoïdes en fonction de leur chromosome X ou Y, et qui sont basées sur la cytométrie de flux.

D'une manière générale, toutes ces solutions techniques de sexage par cytométrie de flux reposent sur le fait que les spermatozoïdes X contiennent environ 4% d'ADN de plus que les spermatozoïdes Y. Ainsi, dans ces solutions techniques de sexage :
(a) on marque les spermatozoïdes avec un intercalant d'ADN fluorescent, généralement désigné fluorochrome ; les spermatozoïdes X, qui sont présumés contenir une plus grande quantité d'ADN absorbent une quantité plus importante de fluorochrome que les spermatozoïdes Y ;
(b) on injecte une solution contenant les spermatozoïdes marqués dans un canal de transport, et le cas échéant on les soumet à une focalisation hydrodynamique de manière à les aligner et les faire circuler dans ce canal de transport,
(c) on excite les spermatozoïdes marqués avec un rayonnement approprié, afin qu'ils émettent un rayonnement lumineux par fluorescence,
(d) on détecte l'intensité du rayonnement lumineux émis par fluorescence (intensité de fluorescence), et
(e) on sélectionne automatiquement chaque spermatozoïde X ou Y en fonction de cette intensité de fluorescence détectée.

Une première méthode de tri connue (étape (e) susvisée) consiste à soumettre la solution contenant les spermatozoïdes marqués à des vibrations au moyen d'un transducteur piézoélectrique, de manière à former des microgouttelettes, qui dans la mesure du possible ne contiennent chacune qu'un seul spermatozoïde, puis à charger électriquement chaque gouttelette en fonction de l'intensité de fluorescente détectée, et enfin à faire passer successivement les microgouttelettes chargées électriquement dans un champ électrique permettant de séparer les gouttelettes chargées contenant un spermatozoïde X des gouttelettes chargées contenant un spermatozoïde Y.

Cette méthode de tri est décrite par exemple dans les demandes de brevet internationales suivantes: WO2004/104178, WO2004/017041, WO2009/014643, WO2009/151624.

Cette méthode de tri par formation de microgouttelettes présente plusieurs inconvénients. L'étape de formation des microgouttelettes est délicate, et il est difficile de s'assurer qu'une microgouttelette ne contient qu'un seul spermatozoïde. La formation de microgouttelettes est un facteur limitatif pour la cadence de tri. Dans cette méthode, les spermatozoïdes sont soumis à un stress mécanique important qui est susceptible de les endommager de manière irréversible et non contrôlée.

On a proposé dans la demande de brevet internationale WO2010/001254 une autre solution dans laquelle la sélection des spermatozoïdes est effectuée au moyen d'un rayonnement électromagnétique, de type faisceau laser, choisi de manière à altérer de manière sélective, en fonction de l'intensité de fluorescente détectée, les spermatozoïdes marqués et transportés en file indienne dans un canal de transport. Cette solution permet avantageusement d'éviter les inconvénients inhérents à la formation de microgouttelettes. Néanmoins cette solution de sélection au moyen d'un laser présente d'autres inconvénients. L'énergie du rayonnement électromagnétique utilisé pour altérer de manière sélective les spermatozoïdes est absorbée de manière prépondérante par le fluide de transport des spermatozoïdes et également par la paroi du canal de transport traversée par le rayonnement électromagnétique, ce qui normalement conduit à devoir utiliser des lasers de forte puissance pour altérer les spermatozoïdes. Or l'utilisation de lasers de forte puissance est un facteur limitatif pour la cadence de sélection, car les temps de commutation d'un laser augmentent avec la puissance du laser. De plus, l'alignement du laser et de son optique de focalisation par rapport au canal de transport est délicat et très sensible, et le moindre désalignement optique peut nuire de manière importante à l'efficacité du laser. A la connaissance de la demanderesse, cette solution technique n'est pas exploitée commercialement à ce jour.

On a par ailleurs proposé des solutions de tri mettant en oeuvre des rayonnements électromagnétiques qui permettent de dévier sélectivement, en fonction de l'intensité de fluorescente détectée, la trajectoire des spermatozoïdes marqués et alignés dans un canal de transport, ce qui permet d'aiguiller automatiquement les spermatozoïdes en fonction de leur chromosome X ou Y, sans altérer les spermatozoïdes et sans devoir former des microgouttelettes. Ce type de solution nécessite de mettre en oeuvre des rayonnements électromagnétiques de forte puissance pour modifier de manière sélective la trajectoire des spermatozoïdes, et peut être mise en oeuvre uniquement avec une très faible vitesse de transport des spermatozoïdes et avec une très faible cadence de tri. A la connaissance de la demanderesse, cette solution technique n'est pas exploitée commercialement à ce jour et la faisabilité technique de ce type de solution reste à démontrer.

Les solutions techniques susvisées peuvent également être utilisées pour sélectionner in vitro tout autre type connu de cellules eucaryotes.

Il existe donc a à ce jour un besoin de trouver une solution technique de sélection de cellules eucaryotes, et en particulier de spermatozoïdes de mammifères, notamment de spermatozoïdes d'animaux, qui soit efficace, et qui pallie les inconvénients susvisés des solutions de l'art antérieur.

### Objectif de l'invention

La présente invention a pour objectif de proposer une nouvelle solution technique de sélection de cellules eucaryotes qui pallie notamment les inconvénients susvisés des solutions de l'art antérieur.

### Résumé de l'invention

L'invention a ainsi pour premier objet un dispositif permettant de sélectionner cellules eucaryotes, et notamment des spermatozoïdes, tel que défini dans la revendication 1.

Le terme « *altérer* » ou « altération » signifie dans le présent texte que la cellule eucaryote est modifiée directement ou indirectement par le rayonnement électromagnétique d'altération, de sorte que sa viabilité ou motilité est détériorée de manière suffisante pour que la cellule eucaryote ne soit plus viable ou fertile, indépendamment du phénomène physique et/ou biologique et/ou chimique occasionnant cette détérioration. Dans le cadre général de l'invention, cette altération par le rayonnement électromagnétique peut par exemple découler d'une lésion ou altération photochimique de la cellule eucaryote et/ou d'un effet de stress thermique subi par la cellule eucaryote.

Grâce à la combinaison d'un canal de transport microfluidique, et d'une fibre optique intégrée, le rayonnement électromagnétique d'altération peut être délivré dans le canal de transport au plus près de la cellule eucaryote, ce qui permet avantageusement d'améliorer l'interaction du rayonnement électromagnétique avec les cellules eucaryotes, et de ce fait permet avantageusement de mettre en oeuvre une source de rayonnement électromagnétique de plus faible puissance, pouvant être plus rapidement commutée ou modulée. En outre, dans l'invention, l'extrémité d'émission de la fibre optique étant insérée dans ledit passage traversant, sans être en saillie dans le canal de transport, le flux des cellules eucaryotes n'est pas perturbé, ce qui permet de maintenir une localisation précise des cellules eucaryotes dans le canal de transport. Enfin, l'extrémité d'émission de la fibre optique étant insérée dans ledit passage traversant, le dispositif peut être manipulé sans risque de désalignement optique, et est ainsi robuste.

Le dispositif de l'invention peut être utilisé pour sélectionner in vitro des cellules eucaryote, et notamment des spermatozoïdes, et plus particulièrement peut être utilisé dans le domaine de la reproduction animale pour sélectionner in vitro tout type de spermatozoïdes d'animaux, et de manière non limitative et non exhaustive, des spermatozoïdes de bovins, de porcins, d'ovins, d'équidés, de caprins, de lapins, d'oiseaux. L'invention n'est pas limitée à la sélection de spermatozoïdes de type X ou de type Y, mais peut être utilisée pour sélectionner tout autre type de spermatozoïdes.

Plus particulièrement, le dispositif de l'invention peut comporter les caractéristiques additionnelles et facultatives de l'une quelconque des revendications 2 à 12, prises isolément ou en combinaison les unes avec les autres.

L'invention a pour autre objet un procédé de sélection in vitro de cellules eucaryotes pouvant avoir des types différents permettant de les répertorier en au moins deux catégories distinctes. Plus particulièrement, mis non exclusivement les cellules eucaryotes peuvent par exemple se différencier grâce l'ADN de leur noyau. Pour la mise en oeuvre de ce procédé, on utilise le dispositif de sélection susvisé, et on injecte, dans le canal de transport du dispositif de sélection, une solution contenant les cellules eucaryotes à sélectionner, on fait circuler dans le canal de transport lesdites cellules eucaryotes les unes derrière les autres, on détecte automatiquement le type de chaque cellule eucaryote circulant dans le canal de transport, et on irradie de manière sélective, avec le rayonnement électromagnétique d'altération, les cellules eucaryotes qui ont été détectés comme étant d'un même type prédéfini, de manière à les altérer suffisamment pour les rendre non viables, les autres cellules eucaryotes n'étant pas altérées au moyen du rayonnement électromagnétique d'altération.

Préalablement à la mise en oeuvre du procédé de sélection, les cellules eucaryotes peuvent avoir subi pour leur conservation un traitement de congélation ou de refroidissement. Egalement, les cellules eucaryotes peuvent indifféremment avoir été purifiées ou non.

Plus particulièrement, le procédé de l'invention peut comporter les caractéristiques additionnelles et facultatives de l'une quelconque des revendications 15 ou 16, prises isolément ou en combinaison les unes avec les autres.

### Brève description des figures

Les caractéristiques et avantages de l'invention apparaîtront plus clairement à la lecture de la description détaillée ci-après de plusieurs variantes de réalisation de l'invention, laquelle description est donnée à titre d'exemple non limitatif et non exhaustif de l'invention, et en référence aux dessins annexés sur lesquels :
- la figure 1 est représentation schématique d'une première variante de réalisation d'un dispositif de sélection de l'invention, la puce microfluidique de ce dispositif de sélection étant représentée en vue de dessus ;
- la figure 2 est une vue en coupe transversale de la puce microfluidique de ce dispositif de sélection, dans le plan de coupe II-II de la figure 1 ;
- la figure 3 est une vue en coupe transversale de ce dispositif de tri, dans le plan de coupe III-III de la figure 1 ;
- la figure 4 est une vue en coupe transversale de ce dispositif de tri, dans le plan de coupe IV-IV de la figure 3 ;
- la figure 5 est une vue en coupe transversale de ce dispositif de tri, dans le plan de coupe V-Vde la figure 1 ;
- la figure 6 est une vue en coupe transversale de ce dispositif de tri, dans le plan de coupe VI-VI de la figure 1 ;
- la figure 7 est une vue en coupe transversale de ce dispositif de tri, dans le plan de coupe VII-VII de la figure 6 ;
- la figure 8 est une vue en coupe transversale d'une deuxième variante d'un dispositif de sélection avec fibre optique de type « wedge », dans le même plan de coupe que la figure 7 ;
- la figure 9 représente la partie distale d'une fibre optique de type « wedge »,
- la figure 10 est une vue en coupe transversale d'une troisième variante d'un dispositif de sélection avec fibre optique de type « tapered», dans le même plan de coupe que la figure 7 ;
- la figure 11 est une représentation schématique d'une quatrième variante de réalisation d'un dispositif de tri de l'invention, la puce microfluidique de ce dispositif de sélection étant représentée en vue de dessus ;
- la figure 12 est une vue en coupe transversale de la puce microfluidique d'une cinquième variante d'un dispositif de sélection.

### Description détaillée

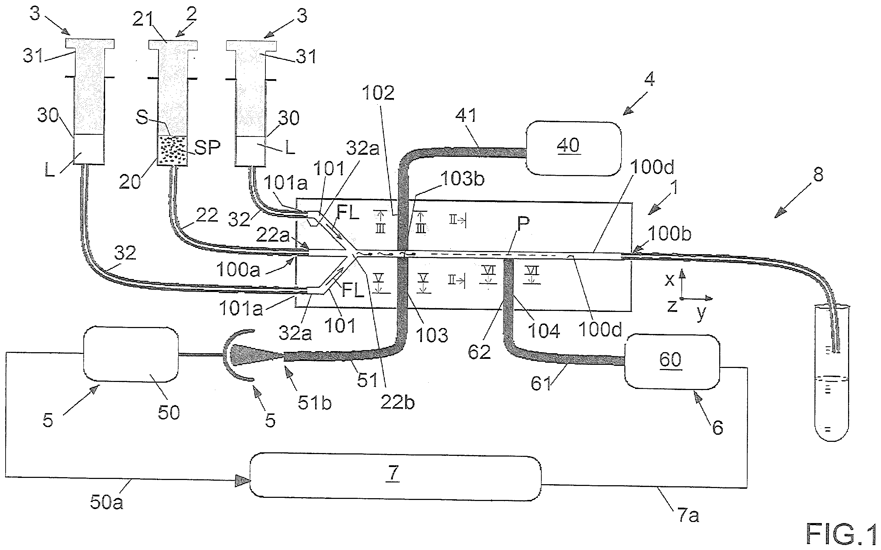

On a représenté sur la figure 1, une première variante de réalisation d'un dispositif permettant la sélection de spermatozoïdes en fonction de leur chromosome, et par exemple de leur chromosome X ou Y.

La description détaillée ci-après est axée sur la sélection de spermatozoïdes se différenciant par leur type de chromosomes. L'invention n'est toutefois pas limitée à la section de spermatozoïdes, mais peut s'appliquer plus généralement à la sélection de cellules eucaryotes pouvant avoir des types différents permettant de les répertorier en au moins deux catégories distinctes. Plus particulièrement, mis non exclusivement les cellules eucaryotes peuvent par exemple se différencier grâce l'ADN de leur noyau.

Le dispositif de la figue 1 comporte :
- une puce microfluidique 1 comportant un canal de transport microfluidique 100 principal,
- des moyens d'injection 2 permettant d'injecter, dans le canal de transport microfluidique 100 principal, une solution S contenant un échantillon de spermatozoïdes SP à sélectionner,
- des moyens (3, 101) de focalisation hydrodynamique permettant d'injecter, dans le canal de transport microfluidique 100 principal, un liquide L de manière à obtenir une focalisation hydrodynamique des spermatozoïdes dans le canal de transport microfluidique 100 principal,
- des moyens 4 d'excitation de la fluorescence des spermatozoïdes SP circulant dans le canal microfluidique 100 principal,
- des moyens 5 de détection de la fluorescence émise par les spermatozoïdes circulant dans le canal de transport microfluidique 100 principal,
- des moyens de sélection 6, comportant une source de rayonnement électromagnétique 60, et permettant d'altérer de manière sélective les spermatozoïdes circulant dans le canal de transport microfluidique principal 100, au moyen du rayonnement électromagnétique émis par la source 60,
- des moyens électroniques de commande 7, qui permettent de commander automatiquement ladite source de rayonnement électromagnétique 60, à partir de la détection de fluorescence réalisée par les moyens de détection 5,
- des moyens de collecte 8 permettant de collecter tous les spermatozoïdes en sortie du canal de transport microfluidique principal 100.

### Puce microfluidique 1- Canal de transport microfluidique principal 100

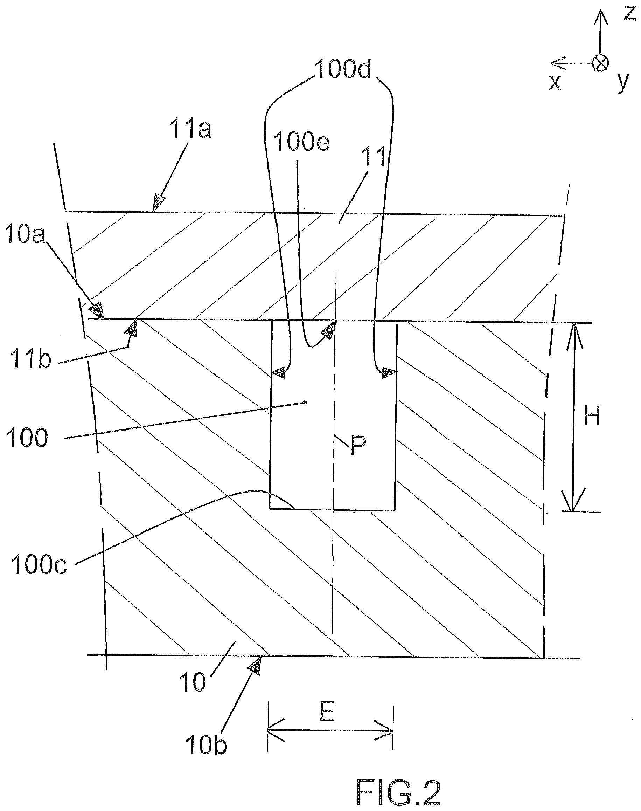

En référence à la figure 2, la puce microfluidique 1 comprend un substrat rigide 10, ayant une face avant 10a et une face arrière 10b, et une plaque 11 fixée contre la face avant 10a du substrat 10.

En référence à la figure 1, dans cette variante particulière de réalisation, le canal de transport microfluidique principal 100 est rectiligne et s'étend suivant la direction longitudinale Y correspondant à la direction de déplacement des spermatozoïdes dans le canal microfluidique 100. Ce canal microfluidique 100 traverse de part en part le substrat 10 et comporte une ouverture d'admission 100a et une ouverture d'évacuation 100b.

Dans une autre variante, tout ou partie de ce canal microfluidique principal 100 pourrait ne pas être rectiligne.

Plus particulièrement, en référence à la figure 2, la section transversale (dans un plan (X,Z)) du canal de transport microfluidique principal 100 est rectangulaire, de hauteur H mesurée dans la direction Z perpendiculaire à la face supérieure 10a plane du substrat 10, et de largeur E mesurée dans la direction transversale X, parallèle à la face supérieure 10a plane du substrat 10 et perpendiculaire à la direction longitudinale Y du canal microfluidique. La hauteur H du canal 100 est supérieure à la largeur E du canal microfluidique100.

La largeur E du canal est inférieure à 1mm, et plus préférentielle encore inférieure à 100µm. La hauteur H du canal peut également être inférieure à 1mm.

La structure et la technique de fabrication de la puce microfluidique 1 comportant ledit canal de transport microfluidique 100 sont sans importance et ne sont pas limitatives de l'invention.

A titre d'exemple uniquement, dans la variante de réalisation de la figure 2, le canal microfluidique principal 100 est délimité par une rainure ayant une forme en U en section transversale et réalisée dans la face supérieure 10a du substrat 10, et par la face arrière 11b de la plaque 11. La paroi de fond 100c de la rainure forme la paroi de fond du canal microfluidique 100 ; les deux parois 100d de la rainure qui sont parallèles, et qui sont transversales, et plus particulièrement perpendiculaires à la paroi de fond 100c, forment les deux parois longitudinales 100d du canal microfluidique 100 ; la partie de la face inférieure 11b de la plaque 11, située au droit de la rainure en U, forme la paroi supérieure 100e du canal microfluidique 100.

Cette section transversale rectangulaire du canal microfluidique 100 permet, de manière connue en soi, de faciliter l'orientation spatiale des spermatozoïdes dans le canal 100 lors de l'étape de focalisation hydrodynamique. En particulier, les spermatozoïdes ayant une forme non sphérique et aplatie, la mise en oeuvre d'un canal microfluidique 100 à section transversale rectangulaire contribue à une orientation spatiale des spermatozoïdes avec leur face aplatie de plus grande surface orientée sensiblement parallèlement au plan (Y, Z), c'est-à-dire sensiblement parallèle au plan des deux parois longitudinales 100d du canal microfluidique 100. Il revient à l'homme de l'art de sélectionner judicieusement et de manière connue en soi les dimensions du canal microfluidique 100, et notamment le ratio H/E.

Il convient toutefois de souligner que l'invention n'est pas limitée à la mise en oeuvre d'un canal de transport 100 ayant une section transversale rectangulaire, ledit canal de transport 100 pouvant plus généralement avoir une géométrie différente en section transversale, et pouvant par exemple et de manière non limitative et non exhaustive, avoir une forme circulaire, ovale, polygonale.

Le substrat 10 est réalisé dans un matériau, qui est chimiquement inerte. Par exemple, mais non nécessairement, le matériau du substrat 10 est choisi de manière à pouvoir subir une gravure physique ou chimique, par exemple une gravure par plasma. Plus particulièrement, mais de manière non limitative de l'invention, le substrat est par exemple en silicium ou en arséniure de gallium. Dans ce cas, la rainure (100c,100d) de hauteur H et de largeur E est avantageusement réalisée par une gravure anisotrope de la face supérieure 10a du substrat 10. Le substrat avec la rainure (100c,100d) peut également être produit par impression 3D.

La plaque 11 est dans un matériau qui est chimiquement inerte, et peut être opaque ou transparente. La plaque 11 est par exemple en verre ou en plastique. Elle est fixée au substrat 10 par tout moyen, et par exemple par collage anodique ou thermocompression.

### Moyens d'injection 2 des spermatozoïdes dans le canal microfluidique

Les moyens d'injection 2 ont pour fonction d'injecter, dans le canal microfluidique 100 principal, une solution S contenant un échantillon de spermatozoïdes à sélectionner.

Plus particulièrement, dans la variante de la figure 1, ces moyens d'injection 2 comportent une seringue 20, qui est remplie avec une solution S contenant l'échantillon de spermatozoïdes SP à sélectionner, et qui est associée à un système d'injection automatique 21, qui peut par exemple être de type pousse-seringue ou de type pompe péristaltique. La sortie de la seringue 20 est couplée à un tube capillaire 22, dont la partie distale 22a a été insérée dans le canal microfluidique 100 par l'ouverture d'admission 100a de ce canal 100. Le tube capillaire 22 est un tube souple, dont la section transversale est de préférence adaptée à la section du canal microfluidique 100.

Plus particulièrement, le tube capillaire 22 est de préférence fixé au substrat 10, par tout moyen, et notamment par collage.

Les spermatozoïdes SP contenus dans la seringue 20 sont dilués dans la solution tampon S, qui est compatible biologiquement avec les spermatozoïdes, et par exemple dans une solution aqueuse comportant 30g/L de TRIS (trishydroxyméthylaminométhane), 17,25 g/L d'acide citrique monohydraté et 12,5 g/L de fructose dans de l'eau à un pH d'environ 7. De nombreuses autres solutions tampon S connues de l'homme du métier peuvent être utilisées. On pourra sur ce point se référer par exemple à l'enseignement de la demande de brevet internationale WO2004/088283.

Plus particulièrement, l'ADN des spermatozoïdes SP contenus dans la solution tampon S a été marqué, de manière connue en soi, au moyen d'au moins un fluorochrome, qui peut fluorescer lorsqu'il est associé à l'ADN. Parmi les fluorochromes couramment utilisés pour marquer les spermatozoïdes, on peut citer à titre d'exemples non limitatifs et non exhaustifs : les fluorochromes de type bisbenzimide, et notamment les fluorochromes Hoechst (Hoechst 33342, Hoechst 33258, ...), le bromure d'éthidium, les fluorochromes SYBR tel que le SYBR-14 .

De nombreux autres fluorochromes connus de l'homme du métier peuvent être utilisés. En particulier, pour de plus amples détails sur la réalisation d'une solution tampon S comportant des spermatozoïdes marqués au moyen de fluorochromes, on pourra se référer par exemple à l'enseignement de la demande de brevet internationale WO2004/088283.

En fonctionnement, le système d'injection 21 pousse la solution tampon S contenant les spermatozoïdes SP, de manière à la faire sortir par l'ouverture distale 22b du capillaire 22, et à l'injecter dans le canal microfluidique 100 avec un débit contrôlé automatiquement, et de préférence constant. On a pu vérifier que cette injection de la solution tampon S contenant les spermatozoïdes SP dans le canal microfluidique 100 n'affectait pas la fertilité, et notamment la motilité des spermatozoïdes.

### Moyens (3, 101) de focalisation hydrodynamique des spermatozoïdes

Afin de permettre la mise en oeuvre d'une focalisation hydrodynamique des spermatozoïdes SP dans le canal microfluidique 100, la puce microfluidique 1 comporte deux canaux microfluidiques secondaires 101, qui de manière usuelle sont formés de part et d'autre du canal de transport microfluidique 100 (Figure 1). Chaque canal microfluidique secondaire 101 comporte une ouverture d'admission 101a et débouche, à l'opposé de l'ouverture d'admission 101a, latéralement dans le canal microfluidique principal 100.

La sortie du tube capillaire 22 est positionnée en amont de la zone de jonction entre les canaux microfluidiques secondaires 101 et le canal de transport microfluidique 100, la partie distale 22a du tube capillaire 22 pouvant être insérée plus ou moins profondément dans le canal de transport 100.

Plus particulièrement, et de manière similaire à ce qui a été décrit précédemment pour le canal microfluidique principal 100, chaque canal microfluidique secondaire 101 est délimité d'une part par une rainure en U gravée dans la face supérieure du substrat 10, et d'autre part par la face inférieure 11b de la plaque 11.

Les moyens de focalisation hydrodynamique comportent pour chaque canal secondaire 101, des moyens d'injection 3 sous la forme d'une seringue 30, qui est remplie avec une solution L, et qui est associée à un système d'injection 31 automatique, par exemple de type pousse-seringue ou de type pompe péristaltique. La sortie de la seringue 30 est couplée à un tube capillaire 32, dont la partie distale 32a a été insérée dans le canal microfluidique 100 par l'ouverture d'admission 101 a d'un canal secondaire 101. Chaque tube capillaire 32 est un tube souple, dont la section transversale est de préférence adaptée à la section du canal secondaire 101. Plus particulièrement, chaque tube capillaire 32 est de préférence fixé au substrat 10, par tout moyen, et notamment par collage.

Le liquide utilisé pour les solutions L est de préférence, mais non nécessairement, identique à celui utilisé pour la solution tampon S contenant les spermatozoïdes SP.

En fonctionnement, chaque système d'injection 31 pousse chaque solution L de manière à l'injecter dans le canal microfluidique secondaire 101 correspondant avec un débit contrôlé automatiquement, et de préférence constant.

De manière connue en soi, les débits de chaque solution L et de la solution tampon S contenant les spermatozoïdes SP sont contrôlés automatiquement, de manière à créer dans le canal microfluidique 100a, deux flux laminaires FL à vitesse élevée formés par chaque solution L, de part et d'autre du flux central plus lent formé par la solution S contenant les spermatozoïdes SP. Ces flux laminaires FL permettent, de manière connue, d'entraîner les spermatozoïdes SP dans le canal microfluidique principal 100 en leur faisant subir une focalisation hydrodynamique, de type 2D, qui aboutit sensiblement à aligner les spermatozoïdes SP les uns derrière les autres, avec un espacement sensiblement constant entre deux spermatozoïdes voisins, et avec un alignement des spermatozoïdes SP sensiblement dans un plan P longitudinal parallèle au plan (Y,Z).

La position de ce plan de focalisation hydrodynamique P des spermatozoïdes entre les deux parois longitudinales 100d du canal 100 dépend notamment de la différence de vitesse entre les deux flux laminaires FL de liquide L. Lorsque ces vitesses sont égales, le plan de focalisation hydrodynamique P des spermatozoïdes est sensiblement centré entre les deux parois longitudinales 100d du canal 100 (Figure2). Dans le cadre de l'invention, le plan de focalisation hydrodynamique P des spermatozoïdes peut être décentré par rapport aux deux parois longitudinales 100d du canal 100.

L'invention n'est pas limitée à une focalisation hydrodynamique 2D des spermatozoïdes SP dans le canal microfluidique principal 100. Il est également possible dans le cadre de l'invention de mettre en oeuvre une focalisation hydrodynamique 3D, tel que décrit par exemple dans la demande de brevet internationale WO2011/005776, de manière à aligner les spermatozoïdes sensiblement le long d'un axe de focalisation hydrodynamique longitudinal parallèle à l'axe Y du canal microfluidique 100.

### Moyens 4 d'excitation de la fluorescence

Les moyens 4 d'excitation de la fluorescence comportent une source 40 de rayonnement électromagnétique, de type source laser, dont la longueur d'onde est adaptée au marqueur (fluorochrome) des spermatozoïdes. Par exemple, et de manière non limitative de l'invention, on met en oeuvre un rayonnement électromagnétique d'excitation de longueur d'onde comprise entre 300nm et 400nm, et par exemple plus particulièrement aux alentours de 375 nm lorsque les spermatozoïdes SP ont été marqués avec du Hoechst.

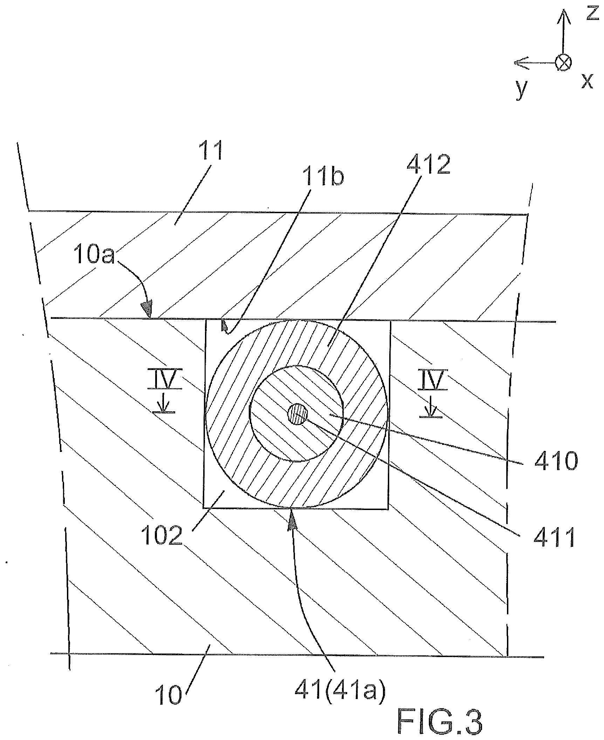

La puce microfluidique 1 comporte un premier passage traversant 102, qui débouche dans le canal microfluidique principal 100 (figures 1 et 3) dans une partie du canal microfluidique principal 100 située en aval de la zone de focalisation hydrodynamique (zone de de jonction entre les canaux 100 et 101).

Dans la variante particulière de la figure 1, ce passage traversant 102 est réalisé à travers l'une des parois longitudinales 100d du canal microfluidique 100.

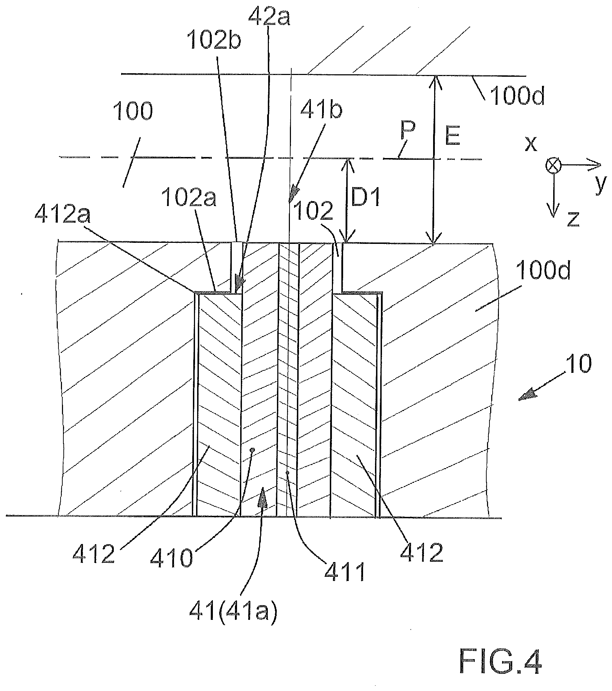

Dans la variante illustrée sur les figures 1 et 4, la distance entre la sortie 102b dans le canal de transport 100 du passage traversant 102 et la paroi opposée 100d du canal de transport 100 correspond à la largeur E du canal de transport 100, et est de préférence inférieure à 1mm, plus préférentiellement inférieure à 100µm.

Ce passage traversant 102 est délimité par une rainure en U gravée dans la face supérieure 10a du substrat 10 et par la face inférieure 11b de la plaque 11. Dans une autre variante, le passage traversant 101 pourrait être percé à travers le substrat 10.

La sortie de la source de rayonnement électromagnétique 40 est couplée à une fibre optique 41 dont la partie distale 41a est insérée dans ce premier passage traversant 102, de telle sorte que l'extrémité d'émission 41b (figure 4) de la fibre optique 41 ne soit pas en saillie dans le canal microfluidique 100, afin de ne pas perturber les écoulements de fluides dans le canal microfluidique 100, et de ce fait de ne pas perturber le positionnement et l'orientation spatiale des spermatozoïdes SP circulant dans le canal microfluidique 100. De préférence, l'extrémité d'émission 41b de la fibre optique 41 est positionnée au plus près de ce canal microfluidique 100, et de préférence affleure le canal microfluidique 100.

Sur cette figure 4, la gaine mécanique de la fibre optique est référencée 412, la gaine optique de la fibre optique est référencée 410, et le coeur de la fibre optique est référencée 411. Dans cette variante, l'extrémité distale de la gaine optique 410 et du coeur 411de la fibre optique, par laquelle est émis le rayonnement électromagnétique d'excitation, est dénudée. Dans une autre variante, l'extrémité distale de la gaine optique 410 et du coeur 411 de la fibre optique pourrait ne pas être dénudée et être entourée par la gaine mécanique 412 de la fibre optique 41.

Plus particulièrement, dans la variante de la figure 4, le passage traversant 102 est profilé de manière à comporter un épaulement 102a formant une butée de positionnement 102a pour l'extrémité distale 412a de la gaine mécanique 412 de la fibre optique. Il suffit ainsi d'insérer la fibre optique jusqu'à ce que l'extrémité distale 412a de la gaine mécanique 412 soit bloquée par la butée de positionnement 102a, ce qui permet avantageusement un positionnement simple et précis de l'extrémité distale d'émission de la fibre optique 41 par rapport au canal microfluidique 100.

En fonctionnement, lorsque l'extrémité d'émission de la fibre optique 41 affleure le canal de transport 100, le rayonnement électromagnétique d'excitation est émis par la fibre optique 41 directement dans le canal de transport 100. Lorsque l'extrémité d'émission de la fibre optique 41 est positionnée légèrement en retrait dans le passage traversant 102, le rayonnement électromagnétique d'excitation est émis dans ce passage traversant 102 puis pénètre dans le canal de transport 100.

Cette insertion de la fibre optique 41 dans la puce microfluidique 1, à proximité du canal microfluidique 100, permet avantageusement d'amener le rayonnement électromagnétique d'excitation au plus près des spermatozoïdes SP circulant dans le canal microfluidique, ce qui contribue à améliorer les performances de l'excitation de la fluorescence.

Afin également d'améliorer les performances de l'excitation de la fluorescence, la distance D₁ (figure 4), entre la sortie 102b dans le canal de transport 100 du premier passage traversant 102 et le plan de focalisation hydrodynamique P (focalisation hydrodynamique 2D) ou l'axe de focalisation hydrodynamique (focalisation hydrodynamique 3D) des spermatozoïdes SP dans le canal de transport 100, est de préférence très faible. On réduit ainsi avantageusement la longueur du trajet du rayonnement électromagnétique d'excitation jusqu'aux spermatozoïdes SP, à travers le liquide transportant les spermatozoïdes dans le canal de transport 100. De préférence, mais non nécessairement cette distance D₁ est inférieure à 1mm, plus préférentiellement inférieure à 100µm, et plus préférentiellement encore inférieure à 50µm.

L'insertion de la fibre optique 41 dans la puce microfluidique 1 permet également d'éviter les risques de désalignement du rayonnement électromagnétique d'excitation par rapport au canal microfluidique 100 lorsque l'on manipule la puce microfluidique 1.

### Moyens 5 de détection de la fluorescence

Les moyens 5 de détection de la fluorescence comportent une fibre optique 51 et un photo-détecteur 50, de type photomultiplicateur (PM) qui est adapté pour détecter la longueur d'onde de fluorescence des spermatozoïdes, à savoir par exemple une longueur d'onde comprise entre 400nm et 500nm, et par exemple aux alentours de 460nm lorsque les spermatozoïdes ont été marqués avec un fluorochrome de type Hoechst.

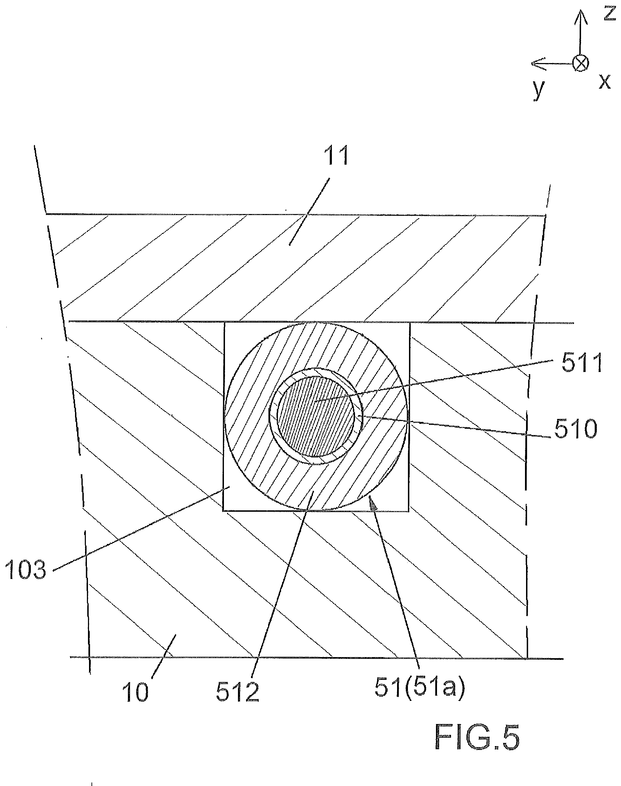

La puce microfluidique 1 comporte un deuxième passage traversant 103, qui débouche dans le canal microfluidique principal 100 (figures 1 et 5) en vis à vis du premier passage traversant 103. Dans la variante particulière de la figure 1, ce passage traversant 103 est réalisé à travers l'autre paroi longitudinale 100d du canal microfluidique 100.

La distance entre la sortie 103b dans le canal de transport 100 du passage traversant 103 et la paroi opposée 100d du canal de transport 100 correspond à la largeur E du canal de transport 100, et est de préférence inférieure à 1mm, plus préférentiellement inférieure à 100µm.

Sur cette figure 5, la gaine mécanique de la fibre optique est référencée 512, la gaine optique de la fibre optique est référencée 510, et le coeur de la fibre optique est référencée 511.

De manière identique à ce qui a été précédemment décrit pour la fibre optique 41, la partie distale 51a de la fibre optique de détection 51 est insérée dans ce deuxième passage traversant 103de telle sorte que l'extrémité distale de la fibre optique 51 ne soit pas en saillie dans le canal microfluidique 100, et est de préférence positionnée au plus près de ce canal microfluidique 100.

L'extrémité d'émission 51b de la fibre de détection 51 est positionnée en vis à vis du photo-détecteur 50, de telle sorte que la lumière (fluorescence) qui est émise dans le canal microfluidique 100, et qui est captée par la fibre 51, est détectée par le détecteur de lumière 50. Le détecteur de lumière 50 délivre un signal électrique 50a caractéristique de l'intensité lumineuse de la fluorescence qui est détectée.

Cette insertion de la fibre optique de détection 51 dans la puce microfluidique 1, à proximité du canal microfluidique 100, permet avantageusement d'améliorer la détection de fluorescence et contribue à une meilleure discrimination entre un spermatozoïde X et un spermatozoïde Y.

Afin également d'améliorer les performances de la détection de fluorescence, la distance entre la sortie 103b dans le canal de transport 100 du deuxième passage traversant 103 (figure 1-ouverture 103b de ce passage 103 débouchant dans le canal de transport 100) et le plan de focalisation hydrodynamique P (focalisation hydrodynamique 2D) ou l'axe de focalisation hydrodynamique (focalisation hydrodynamique 3D) des spermatozoïdes dans le canal de transport 100 est de préférence très faible. On réduit ainsi avantageusement la longueur du trajet du rayonnement de fluorescence, à travers le liquide transportant les spermatozoïdes dans le canal de transport 100. De préférence, mais non nécessairement cette distance est inférieure à 1mm, plus préférentiellement inférieure à 100µm, et plus préférentiellement encore inférieure à 50µm.

Plus particulièrement, afin de collecter un maximum de lumière, la fibre optique de détection 51 peut par exemple être une fibre optique large coeur 511 (figure 5) contrairement à la fibre optique d'excitation 41, dont le coeur 411 (figure 4) est de plus faible diamètre de manière à concentrer spatialement le rayonnement électromagnétique.

### Moyen de sélection 6

Les moyens de sélection 6 comportent une source 60 de rayonnement électromagnétique, de type source laser (pulsé ou continu) et une fibre optique 61. La sortie de cette source de rayonnement électromagnétique 61 est couplée à la fibre optique 61.

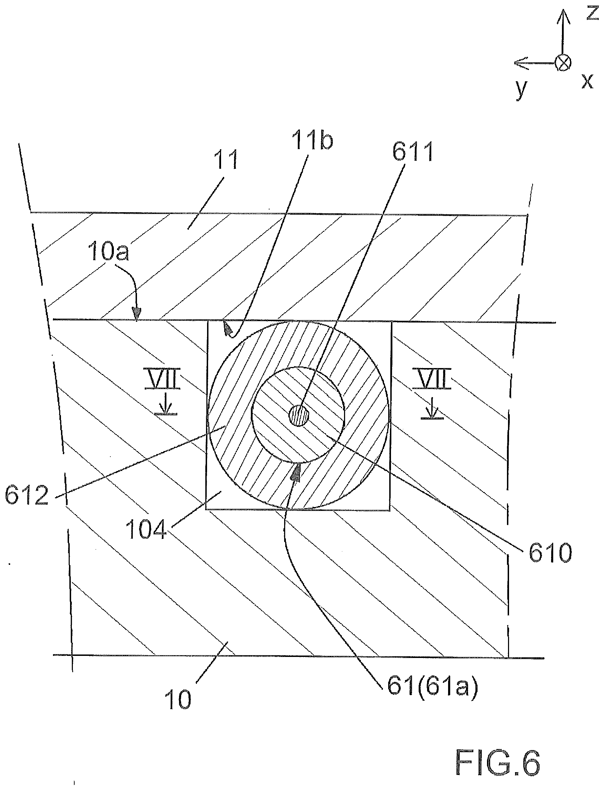

La puce microfluidique 1 comporte un troisième passage traversant 104, qui débouche dans le canal microfluidique principal 100 (figures 1 et 6) dans une partie du canal microfluidique principal 100 située en aval de la zone de détection de fluorescence.

Dans la variante particulière de la figure 1, ce passage traversant 104 est réalisé à travers l'une des parois longitudinales 100d du canal microfluidique 100.

La distance entre la sortie 104b dans le canal de transport 100 du passage traversant 104 et la paroi opposée 100d du canal de transport 100 correspond à la largeur E du canal de transport 100, et est de préférence inférieure à 1mm, plus préférentiellement inférieure à 100µm.

Sur la figure 6, la gaine mécanique de la fibre optique est référencée 612, la gaine optique de la fibre optique est référencée 610, et le coeur de la fibre optique est référencé 611.

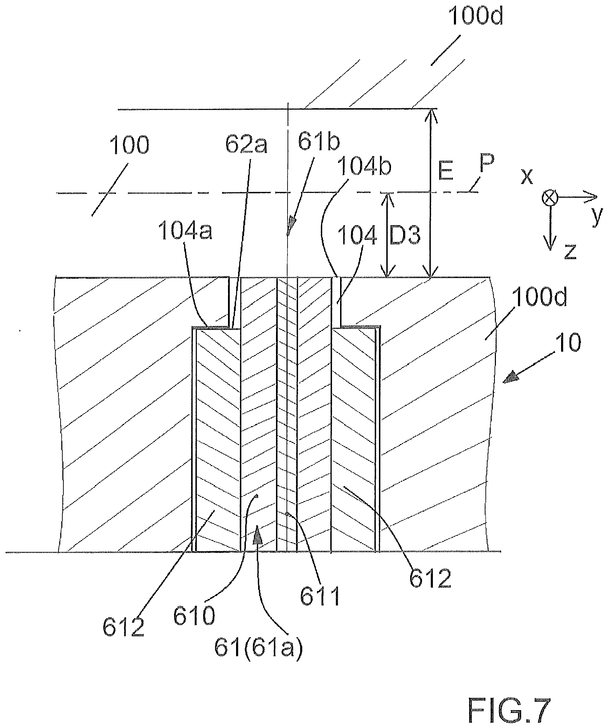

De manière identique à ce qui a été précédemment décrit pour la fibre optique 41, la partie distale 61a de la fibre optique 61 est insérée dans ce troisième passage traversant 104, de telle sorte que l'extrémité distale d'émission 61b (figure 7) de la fibre optique 61 ne soit pas en saillie dans le canal microfluidique 100, et est de préférence positionnée au plus près de ce canal microfluidique 100.

Lorsqu'elle activée, la source 60 émet dans le canal microfluidique 100 un rayonnement électromagnétique d'altération R, qui a pour fonction d'altérer directement ou indirectement un spermatozoïde SP circulant dans le canal microfluidique principal 100 et passant à travers ce rayonnement électromagnétique d'altération, de manière à ce que ce spermatozoïde ne soit plus fertile. Le spermatozoïde SP traversant le rayonnement électromagnétique d'altération est modifié, de sorte que sa viabilité ou motilité est détériorée de manière suffisante pour que le spermatozoïde ne soit plus fertile, indépendamment du phénomène physique et/ou biologique et/ou chimique occasionnant cette détérioration. Dans le cadre général de l'invention, cette altération par le rayonnement électromagnétique peut par exemple découler d'une lésion ou altération photochimique du spermatozoïde et/ou d'un effet de stress thermique subi par le spermatozoïde.

Pour obtenir cette altération, on peut pratiquer une longueur d'onde pour le rayonnement électromagnétique d'altération qui est sélectionnée dans une large gamme spectrale. Plus particulièrement, mais non exclusivement, la longueur d'onde du rayonnement électromagnétique d'altération sera typiquement sélectionnée dans une gamme de longueur d'ondes allant de l'UV à l'Infrarouge.

L'insertion de la fibre optique 61 dans la puce microfluidique 1, à proximité du canal microfluidique 100, permet avantageusement d'amener le rayonnement électromagnétique d'altération des spermatozoïdes au plus près des spermatozoïdes SP circulant dans le canal microfluidique.

En fonctionnement, lorsque l'extrémité d'émission de la fibre optique 61 affleure le canal de transport 100, le rayonnement électromagnétique d'altération est émis par la fibre optique 61 directement dans le canal de transport 100. Lorsque l'extrémité d'émission de la fibre optique 61 est positionnée légèrement en retrait dans le passage traversant 104, le rayonnement électromagnétique d'altération est émis dans ce passage traversant 102 puis pénètre dans le canal de transport 100.

Afin également d'améliorer les effets du rayonnement électromagnétique d'altération, la distance D₃ (figure 7), entre la sortie 104b dans le canal de transport 100 du troisième passage traversant 104 et le plan de focalisation hydrodynamique P (focalisation hydrodynamique 2D) ou l'axe de focalisation hydrodynamique (focalisation hydrodynamique 3D) des spermatozoïdes SP dans le canal de transport 100, est de préférence très faible. On réduit ainsi avantageusement la longueur du trajet du rayonnement électromagnétique d'altération jusqu'aux spermatozoïdes SP, à travers le liquide transportant les spermatozoïdes dans le canal de transport 100. De préférence, mais non nécessairement cette distance D₃ est inférieure à 1mm, plus préférentiellement inférieure à 100µm, et plus préférentiellement encore inférieure à 50µm.

Il en résulte notamment qu'on réduit fortement les phénomènes d'absorption du rayonnement électromagnétique d'altération par le liquide circulant dans le canal microfluidique 100, et on élimine les phénomènes d'absorption du rayonnement électromagnétique d'altération par le substrat 10 de la puce microfluidique, comparativement à une solution dans laquelle le rayonnement électromagnétique d'altération devrait passer à travers ledit substrat. Il devient ainsi possible d'altérer les spermatozoïdes en les irradiant avec un rayonnement électromagnétique de faible puissance, par exemple ayant une puissance moyenne inférieure à 10W, et de préférence inférieur à 1W. L'invention n'est toutefois pas limitée à ces valeurs de puissance.

Grâce à l'intégration de la partie distale 61a de la fibre optique 61 dans la puce microfluidique, on évite également les risques de désalignement du rayonnement électromagnétique d'altération par rapport au canal microfluidique 100, notamment lorsque l'on manipule la puce microfluidique 1.

### Focalisation - fibre « wedge » ou fibre « tapered »

De préférence, la fibre optique 61 est une fibre optique microlentillée dont l'extrémité d'émission 61b est profilée de manière à former une lentille permettant de focaliser le rayonnement électromagnétique d'altération R dans le canal microfluidique 100 par rapport au trajet des spermatozoïdes SP, c'est-à-dire dans le cas des figures annexées dans le plan d'alignement P des spermatozoïdes SP.

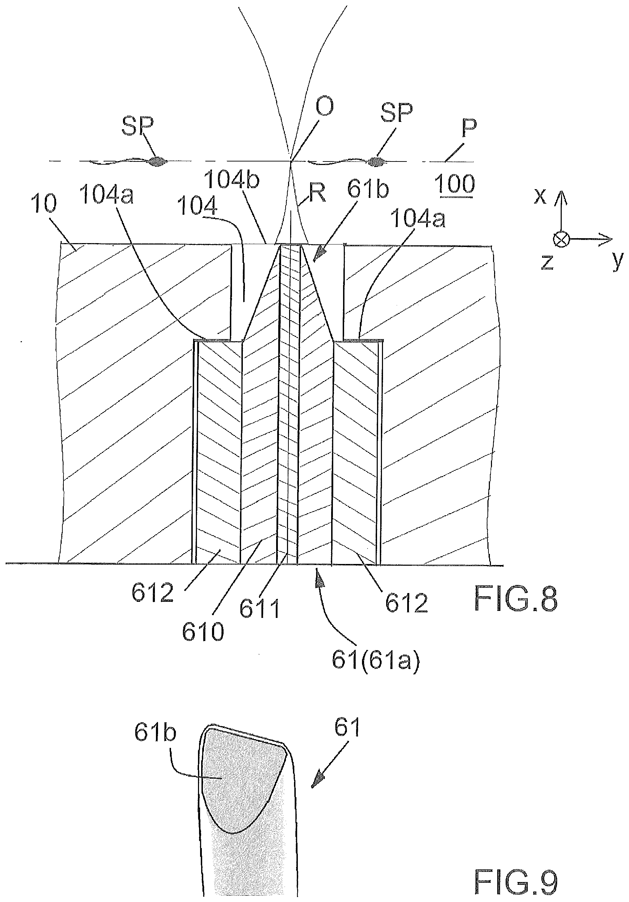

Plus particulièrement, en référence aux figures 8 et 9, dans une variante de réalisation la fibre optique 61 est une fibre optique dite « wedge », c'est-à-dire dont l'extrémité distale d'émission forme une lentille biseautée sur les deux faces suivant une direction.

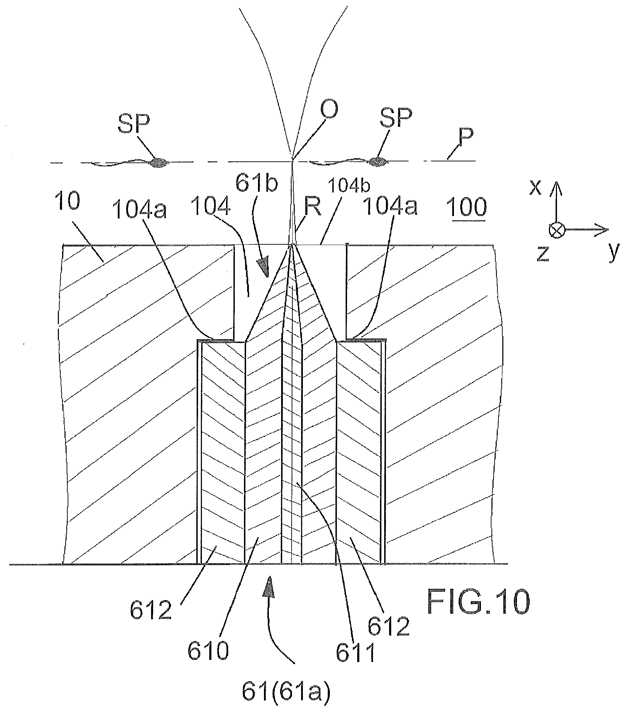

Plus particulièrement, en référence à la figure 10 dans une variante de réalisation la fibre optique 61 est une fibre optique dite « tapered », c'est-à-dire dont l'extrémité distale d'émission forme une lentille conique.

La position de l'extrémité d'émission 61b de la fibre optique 61 par rapport au plan de focalisation hydrodynamique P des spermatozoïdes SP est choisie de manière à optimiser l'interaction entre le rayonnement électromagnétique d'altération F par rapport au plan de focalisation hydrodynamique P des spermatozoïdes SP, en sorte de délivrer le maximum d'énergie sensiblement dans ce plan d'alignement P des spermatozoïdes.

Plus particulièrement, en référence à la figure 8, la fibre optique « wedge » 61 permet une focalisation du rayonnement électromagnétique d'altération R le long sensiblement d'une ligne de focalisation O dans le plan de focalisation hydrodynamique P des spermatozoïdes SP et parallèle à l'axe Z. La fibre optique « tapered » permet une focalisation du rayonnement électromagnétique d'altération R en un point de focalisation O sensiblement dans le plan de focalisation hydrodynamique P des spermatozoïdes SP.

On optimise ainsi l'utilisation de la puissance du rayonnement électromagnétique d'altération R pour obtenir l'altération des spermatozoïdes SP, ce qui permet de réduire la puissance de la source rayonnement électromagnétique 60.

### Moyens électroniques de commande 7

Les moyens électroniques de commande 7 reçoivent en entrée le signal 50a de détection de fluorescence délivré par le photo-détecteur 50, et délivre en sorte un signal de commande 7a permettant de commander automatiquement ladite source de rayonnement électromagnétique 60, à partir de la détection de fluorescence.

Plus particulièrement, les moyens électroniques de commande 7 sont par exemple conçus pour comparer le signal 50a de détection de fluorescence avec un seuil prédéfini, qui de manière connue permet une discrimination entre un spermatozoïde X et un spermatozoïde Y, et de commander automatiquement ladite source de rayonnement électromagnétique 60 :
- de manière à ce que le rayonnement électromagnétique d'altération R soit émis dans le canal microfluidique 100, si on souhaite altérer le spermatozoïde SP qui a été détecté lorsque celui-ci traverse ledit rayonnement électromagnétique d'altération R
   ou
- de manière à ce que le rayonnement électromagnétique d'altération R ne soit pas émis dans le canal microfluidique 100, si on souhaite conserver intact le spermatozoïde SP qui a été détecté.

Dans l'échantillon collecté par les moyens de collecte 8, on obtient ainsi une semence sexée, les spermatozoïdes d'un type donné (par exemple Y) sont ainsi intacts et fertiles et les spermatozoïdes de l'autre type (par exemple X) sont altérés de manière suffisante pour ne plus être fertiles.

L'invention permettant notamment de réduire la puissance de la source de rayonnement électromagnétique, la modulation du rayonnement électromagnétique d'altération R en fonction de la détection de fluorescence peut être très rapide, ce qui permet d'atteindre des cadences de sélection élevées.

A titre d'exemple uniquement, et de manière non limitative, il est par exemple possible de mettre oeuvre l'invention avec une source laser de puissance 60 de l'ordre de quelques centaines de mW, émettant dans une gamme de longueur d'ondes entre 1µm et 3µm, et avec une fibre optique « tapered » 60, et de sélectionner des spermatozoïdes avec une cadence d'un spermatozoïde toutes les 100µs.

### Autres variantes

L'invention n'est pas limitée aux variantes de réalisation susvisées. De manière non limitative et non exhaustive de l'invention, d'autres variantes de réalisation brièvement décrites ci-après peuvent par exemple être également envisagées.

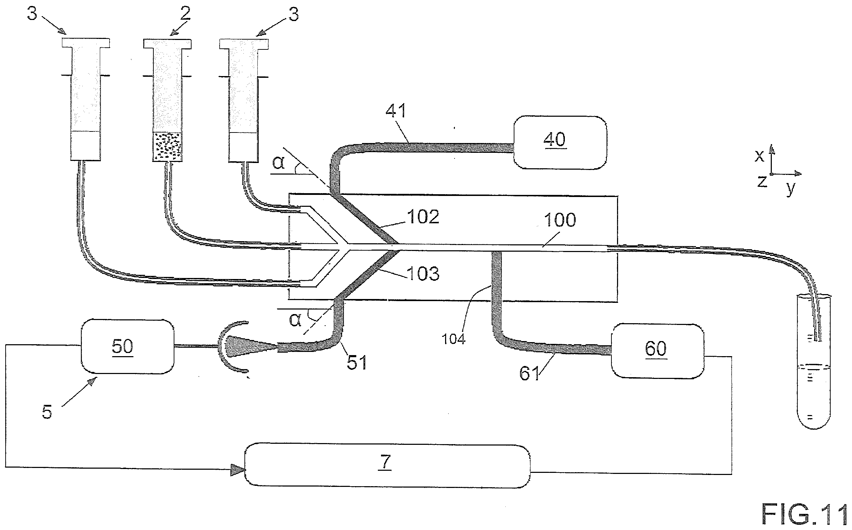

En référence à la variante de la figure 11, les passages traversants 102 et 103 respectivement pour la fibre optique d'excitation 41 et pour la fibre optique de détection de fluorescence 51 ne sont pas nécessairement orientés perpendiculairement à l'axe longitudinal Y du canal microfluidique principal 100, mais peuvent former avec cet axe longitudinal Y un angle α inférieur à 90°.

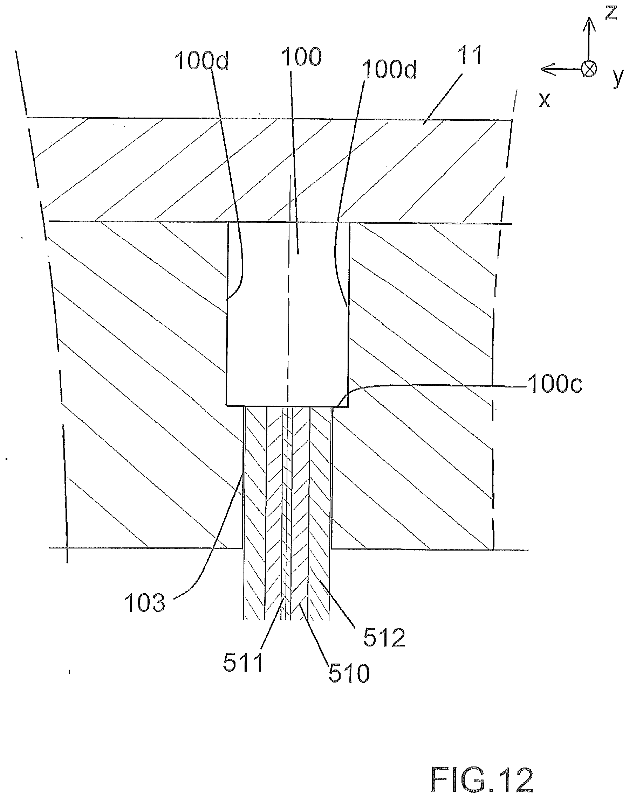

En référence à la variante figure 12, la fibre optique 51 de détection de la fluorescence peut être intégrée à la puce microfluidique 1 en ayant sa partie distale insérée dans un passage traversant 103 réalisé dans la paroi de fond 100c du canal microfluidique 100. Les fibres optiques 41 et 51 sont ainsi avantageusement orientées à angle droit l'une par rapport à l'autre, ce qui permet d'éviter les risques de détection d'une fluorescence parasite qui pourrait notamment être émise par la fibre optique 41 des moyens 4 d'excitation de fluorescence. A l'inverse, dans une autre variante, ce peut être la fibre optique 41 d'excitation de la fluorescence qui peut être intégrée à la puce microfluidique 1 en ayant sa partie distale insérée dans un passage traversant 103 réalisé dans la paroi de fond 100c du canal microfluidique 100.

Dans une autre variante de réalisation (non représentée), les moyens 4 d'excitation de fluorescence peuvent être complétement externes à la puce microfluidique 1 et ne pas comporter de fibre optique intégrée à la puce microfluidique 1.

De la même manière, les moyens 5 de détection de fluorescence peuvent être complètement externes à la puce microfluidique 1 et ne pas comporter de fibre optique intégrée à la puce microfluidique 1.

## Revendications

1. Dispositif permettant de sélectionner des cellules eucaryotes (SP), lequel dispositif comporte un canal de transport (100), dans lequel peut circuler une solution contenant lesdites cellules eucaryotes (SP), un premier passage traversant (104) débouchant dans ledit canal de transport (100), une source de rayonnement électromagnétique (60), qui est couplée à une première extrémité d'une première fibre optique (61), l'autre extrémité d'émission (61b) de la première fibre optique étant insérée dans ledit premier passage traversant (104), sans être en saillie dans le canal de transport (100), le canal de transport (100) étant un canal microfluidique dont au moins une dimension en section transversale est inférieure à 1mm, et plus particulièrement inférieure à 100µm, et ledit dispositif comportant en outre des moyens électroniques de commande (7), qui permettent de commander automatiquement ladite source de rayonnement électromagnétique (60), de manière à altérer de manière sélective les cellules eucaryotes (SP) circulant dans le canal de transport (100), au moyen du rayonnement électromagnétique d'altération (R) émis par la source de rayonnement électromagnétique (60).

2. Dispositif selon la revendication 1, comportant des moyens optiques de focalisation, qui sont fixés ou intégrés à l'extrémité d'émission (61b) de la première fibre optique, et qui permettent de focaliser dans ledit canal de transport (100) le rayonnement électromagnétique (R) émis en sortie de la première fibre optique (61), ou des moyens optiques de focalisation formés par l'extrémité d'émission (61b) de la première fibre optique (61), qui est profilée de manière à focaliser dans ledit canal de transport (100) le rayonnement électromagnétique (R) émis en sortie de la fibre optique (61)

3. Dispositif selon la revendication 2, dans lequel l'extrémité d'émission (61b) de la
première fibre optique (61) est de forme conique, ou dans lequel l'extrémité d'émission (61b) de la fibre première optique (61) est de type « wedge ».

4. Dispositif selon l'une quelconque des revendications précédentes, dans lequel l'extrémité d'émission (61b) de la première fibre optique affleure le canal de transport (100) sans être en saillie dans le canal de transport.

5. Dispositif selon l'une quelconque des revendications précédentes, dans lequel le canal de transport (100) a une section transversale rectangulaire, et de préférence dans lequel la largeur (E) du canal de transport (100) rectangulaire est inférieure à 1mm, et de préférence inférieure à 100µm.

6. Dispositif selon l'une quelconque des revendications précédentes, comportant une deuxième source de rayonnement électromagnétique (40) apte à émettre rayonnement électromagnétique d'excitation, qui permet d'exciter l'émission par fluorescence des cellules eucaryotes (SP) circulant dans ledit canal de transport (100), et au moins un photo-détecteur (50) permettant de détecter la fluorescence émise par lesdites cellules eucaryotes (SP), et dans lequel les moyens électroniques de commande (7) sont aptes traiter un signal électrique de détection (50a) délivré par ledit photo-détecteur (50), et à commander la première source de rayonnement électromagnétique (60), pour l'altération sélective des cellules eucaryotes (SP), en fonction de ce signal de détection (50a).

7. Dispositif selon la revendication 6, comportant un passage traversant (102) pour l'excitation de fluorescence, qui débouche dans ledit canal de transport (100), en amont du premier passage traversant (104), et dans lequel la deuxième source de rayonnement électromagnétique (40) est couplée à une première extrémité d'une fibre optique (41) d'excitation de fluorescence, l'autre extrémité d'émission (41b) de cette fibre optique (41) d'excitation de fluorescence étant insérée dans ledit passage traversant (102) pour l'excitation de fluorescence, sans être en saillie dans le canal de transport (100).

8. Dispositif selon l'une quelconque des revendications 6 à 7, comportant un passage traversant (103) pour la détection de fluorescence, qui débouche dans ledit canal de transport (100) en amont du premier passage traversant (104), et dans lequel une extrémité (41b) d'une fibre optique (51) de détection de fluorescence est insérée dans ce passage traversant (103), sans être en saillie dans le canal de transport (100), et l'autre extrémité (51b) d'émission de ladite fibre optique (51) de détection de fluorescence étant associée au photo-détecteur (50).

9. Dispositif selon la revendication 8, dans lequel la distance (E) entre la sortie (102b) dans le canal de transport (100) du passage traversant (102) pour l'excitation de fluorescence et la paroi opposée du canal de transport (100) est inférieure à 1mm, plus préférentiellement inférieure à 100µm, et/ou dans lequel la distance (E) entre la sortie (103b) dans le canal de transport (100) du passage traversant (103) pour la détection de fluorescence et la paroi opposée du canal de transport (100) est inférieure à 1mm, plus préférentiellement inférieure à 100µm.

10. Dispositif selon l'une quelconque des revendications précédentes, dans lequel la distance (E) entre la sortie (104b) dans le canal de transport (100) du premier passage traversant (104) et la paroi opposée du canal de transport (100) est inférieure à 1mm, plus préférentiellement inférieure à 100µm, et plus préférentiellement encore inférieure à 50µm.

11. Dispositif selon l'une quelconque des revendications précédentes, comportant des moyens (2) permettant d'injecter dans ledit canal de transport (100) une solution (S) contenant des cellules eucaryotes (SP), et de préférence des cellules eucaryotes (SP) marquées au moyen d'au moins un fluorochrome, et comportant des moyens de focalisation hydrodynamique (3 ; 101) permettant d'injecter dans le canal de transport (100) un liquide (L) de manière à entraîner les cellules eucaryotes (SP) dans le canal de transport (100), en les positionnant sensiblement dans un plan de focalisation hydrodynamique (P) ou sensiblement le long d'un axe de focalisation hydrodynamique et en les espaçant les unes derrière les autres.

12. Dispositif selon la revendication 11 et l'une quelconque des revendications 2 à 5, dans lequel lesdits moyens optiques de focalisation permettent de focaliser le rayonnement électromagnétique d'altération (R) sensiblement dans ledit plan (P) de focalisation hydrodynamique des cellules eucaryotes (SP) ou sensiblement sur l'axe de focalisation hydrodynamique des cellules eucaryotes (SP).

13. Utilisation du dispositif visé à l'une quelconque des revendications précédentes pour sélectionner automatiquement in vitro des cellules eucaryotes, plus particulièrement des spermatozoïdes, et plus particulièrement encore des spermatozoïdes d'animaux.

14. Procédé de sélection in vitro de cellules eucaryotes (SP) pouvant avoir des types différents permettant de les répertorier en au moins deux catégories distinctes, au moyen d'un dispositif de sélection visé à l'une quelconque des revendications 1 à 12, au cours duquel on injecte, dans le canal de transport (100) du dispositif de tri, une solution (S) contenant les cellules eucaryotes (SP) à trier, on fait circuler dans le canal de transport 100) lesdites cellules eucaryotes (SP) les unes derrière les autres, on détecte automatiquement le type de chaque cellule eucaryote circulant dans le canal de transport (100), et on irradie de manière sélective, avec le rayonnement électromagnétique d'altération (R), les cellules eucaryotes (SP) qui ont été détectées comme étant d'un même type prédéfini, de manière à les altérer suffisamment pour les rendre non viables, les autres cellules eucaryotes n'étant pas altérées au moyen du rayonnement électromagnétique d'altération (R).

15. Procédé selon la revendication 14, au cours duquel on focalise de manière hydrodynamique les cellules eucaryotes dans le canal de transport de manière à les aligner les unes derrière les autres sensiblement dans un plan de focalisation hydrodynamique (P) ou sensiblement le long d'un axe de focalisation hydrodynamique, et dans lequel la distance (D₃) entre la sortie (104b) dans le canal de transport (100) du premier passage traversant (104) et le plan de focalisation hydrodynamique (P) ou l'axe de focalisation hydrodynamique des cellules eucaryotes (SP) dans le canal de transport (100), est inférieure à 1mm, plus préférentiellement inférieure à 100µm, et plus préférentiellement encore inférieure à 50µm, et/ou dans lequel la distance (D₁), entre la sortie (102b) dans le canal de transport (100) du passage traversant (102) pour l'excitation de fluorescence et ledit plan de focalisation hydrodynamique (P) ou ledit axe de focalisation hydrodynamique des cellules eucaryotes (SP) dans le canal de transport (100), est inférieure à 1mm, plus préférentiellement inférieure à 10µm, et plus préférentiellement encore inférieure à 50µm, et/ou dans lequel la distance entre la sortie (103b) dans le canal de transport (100) du passage traversant (103) pour la détection de fluorescence et le plan de focalisation hydrodynamique (P) ou l'axe de focalisation hydrodynamique des cellules eucaryotes (SP) dans le canal de transport (100), est inférieure à 1mm, plus préférentiellement inférieure à 100µm, et plus préférentiellement encore inférieure à 50µm.

16. Procédé selon l'une quelconque des revendications 14 à 15, dans lequel les cellules eucaryotes sont des spermatozoïdes (SP), notamment des spermatozoïdes d'animaux, pouvant avoir des chromosomes différents.

## Patentansprüche

1. Vorrichtung zum Selektieren von eukaryotischen Zellen (SP), wobei die Vorrichtung einen Transportkanal (100), in dem eine die eukaryotischen Zellen (SP) enthaltende Lösung fließen kann, wobei ein erster Durchgangskanal (104) in den Transportkanal (100) mündet, und eine Quelle elektromagnetischer Strahlung (60) umfasst, die mit einem ersten Ende einer ersten optischen Faser (61) gekoppelt ist, wobei das andere Emissionsende (61b) der ersten optischen Faser in den ersten Durchgangskanal (104) eingeführt wird, ohne in den Transportkanal (100) zu ragen, wobei der Transportkanal (100) ein mikrofluidischer Kanal mit mindestens einer Querschnittsabmessung von weniger als 1 mm und insbesondere weniger als 100 µm ist, und wobei die Vorrichtung ferner elektronische Steuermittel (7) umfasst, die die Quelle der elektromagnetischen Strahlung (60) automatisch steuern, um die im Transportkanal (100) zirkulierenden eukaryontischen Zellen (SP) durch die von der Quelle der elektromagnetischen Strahlung (60) emittierte elektromagnetische Änderungsstrahlung (R) selektiv zu verändern.

2. Vorrichtung nach Anspruch 1, umfassend optische Fokussiermittel, die am Emissionsende (61b) der ersten optischen Faser befestigt oder integriert sind und die es ermöglichen, die am Ausgang der ersten optischen Faser (61) emittierte elektromagnetische Strahlung (R) in den Transportkanal (100) zu fokussieren, oder ein optisches Fokussiermittel, das durch das Emissionsende (61b) der ersten optischen Faser (61) gebildet ist, das ein derartiges Profil hat, dass es die elektromagnetische Strahlung (R), die am Ausgang der optischen Faser (61) emittiert wird, in den Transportkanal (100) fokussiert wird.

3. Vorrichtung nach Anspruch 2, wobei das Emissionsende (61b) der ersten optischen Faser (61) konisch ausgebildet ist oder wobei das Emissionsende (61b) der ersten optischen Faser (61) vom Keiltyp ist.

4. Vorrichtung nach einem der vorhergehenden Ansprüche, wobei das Emissionsende (61b) der ersten optischen Faser bündig mit dem Transportkanal (100) abschließt ohne in den Transportkanal vorzuspringen.

5. Vorrichtung nach einem der vorhergehenden Ansprüche, wobei der der Transportkanal (100) einen rechteckigen Querschnitt aufweist und wobei vorzugsweise die Breite (E) des rechteckigen Förderkanals (100) weniger als 1 mm und vorzugsweise weniger als 100 µm beträgt.

6. Vorrichtung nach einem der vorhergehenden Ansprüche, umfassend eine zweite Quelle elektromagnetischer Strahlung (40), die in der Lage ist, elektromagnetische Anregungstrahlung zu emittieren, die eine Anregung einer Fluoreszenzemission von eukaryontischen Zellen (SP), die in dem Transportkanal (100) zirkulieren, ermöglicht, und und mindestens einen Photodetektor (50) zum Erfassen der von den eukaryotischen Zellen (SP) emittierten Fluoreszenz, und wobei die elektronischen Steuermittel (7) dazu ausgelegt sind, ein von dem Photodetektor (50) geliefertes elektrisches Detektionssignal (50a) zu verarbeiten und die erste Quelle elektromagnetischer Strahlung (60) für die selektive Veränderung der eukaryotischen Zellen (SP) abhängig von diesem Detektionssignal (50a) zu steuern.

7. Vorrichtung nach Anspruch 6, umfassend einen Durchgangskanal (102) zur Fluoreszenzanregung, der stromaufwärts des ersten Durchgangskanals (104) in den Transportkanal (100) mündet, und wobei die zweite Quelle elektromagnetischer Strahlung (40) mit einem ersten Ende einer optischen Fluoreszenzanregungsfaser (41) gekoppelt ist, wobei das andere Emissionsende (41b) der optischen Fluoreszenzanregungsfaser (41) in den Durchgangskanal (102) zur Fluoreszenzanregung eingeführt ist, ohne in den Transportkanal (100) zu ragen.

8. Vorrichtung nach einem der Ansprüche 6 bis 7, umfassend einen Durchgangskanal (103) zur Fluoreszenzdetektion, der stromaufwärts des ersten Durchgangskanals (104) in den Transportkanal (100) mündet, und
wobei ein Ende (41b) einer optischen Fluoreszenzdetektionsfaser (51) in diesen Durchgangskanal (103) eingesetzt wird, ohne in den Transportkanal (100) zu ragen, und das andere Emissionsende (51b) der optischen Fluoreszenzdetektionsfaser (51) dem Photodetektor (50) zugeordnet ist.

9. Vorrichtung nach Anspruch 8, wobei der Abstand (E) zwischen dem Ausgang (102b) des Durchgangskanals (102) für die Fluoreszenzanregung im Transportkanal (100) und der gegenüberliegenden Wand des Transportkanals (100) weniger als 1 mm, vorzugsweise weniger als 100 µm beträgt, und/oder wobei der Abstand (E) zwischen dem Ausgang (103b) des Durchgangskanals (103) zur Fluoreszenzdetektion im Transportkanal (100) und der gegenüberliegenden Wand des Transportkanals (100) weniger als 1 mm, vorzugsweise weniger als 100 µm beträgt.

10. Vorrichtung nach einem der vorhergehenden Ansprüche, wobei der Abstand (E) zwischen dem Ausgang (104b) des ersten Durchgangskanals (104) im Transportkanal (100) und der gegenüberliegenden Wand des Transportkanals (100) weniger als 1 mm, vorzugsweise weniger als 100 µm und vorzugsweise sogar weniger als 50 µm beträgt.

11. Vorrichtung nach einem der vorhergehenden Ansprüche, umfassend Mittel (2) zum Einspritzen einer Lösung (S), die eukaryotische Zellen (SP) und vorzugsweise eukaryotische Zellen (SP), die mit mindestens einem Fluorchrom markiert sind, enthält, in den Transportkanal (100), und umfassend hydrodynamische Fokussiermittel (3; 101) zum Einspritzen einer Flüssigkeit (L) in den Transportkanal (100), um die eukaryotischen Zellen (SP) in den Transportkanal (100) zu spülen, indem sie im Wesentlichen in einer hydrodynamischen Fokussierungsebene (P) oder im Wesentlichen entlang einer hydrodynamischen Fokussierachse in Abständen hintereinander angeordnet sind.

12. Vorrichtung nach Anspruch 11 und einem der Ansprüche 2 bis 5, wobei die optische Fokussiereinrichtung es ermöglicht, die elektromagnetische Änderungsstrahlung (R) im Wesentlichen in der hydrodynamischen Fokussierungsebene (P) der eukaryotischen Zellen (SP) oder im Wesentlichen auf der hydrodynamischen Fokussierungsachse der eukaryotischen Zellen (SP) zu fokussieren.

13. Verwendung der Vorrichtung nach einem der vorhergehenden Ansprüche zum automatischen in-vitro-Selektieren von eukaryotischen Zellen, insbesondere Spermien und insbesondere tierischem Sperma.

14. Verfahren zur in vitro-Selektion von eukaryotischen Zellen (SP), die verschiedene Typen aufweisen können, durch welches diese in mindestens zwei verschiedene Kategorien eingeteilt werden können, mittels einer Vorrichtung zum Selektieren von eukaryotischen Zellen nach einem der Ansprüche 1 bis 12, bei dem eine Lösung (S), die die zu sortierenden eukaryotischen Zellen (SP) enthält, in den Transportkanal (100) der Sortiervorrichtung eingegeben wird, wobei die eukaryotischen Zellen (SP) nacheinander im Transportkanal (100) zirkulieren, der Typ jeder im Transportkanal (100) zirkulierenden eukaryotischen Zelle automatisch erfasst wird und diejenigen eukaryotischen Zellen (SP), für die ein gleicher vorgegebener Typ erfasst wurde, selektiv mit elektromagnetischer Änderungsstrahlung (R) bestrahlt werden, um sie hinreichend verändern, so dass sie nicht mehr lebensfähig sind, wobei die anderen eukaryotischen Zellen nicht durch elektromagnetische Änderungsstrahlung (R) verändert werden.

15. Verfahren nach Anspruch 14, bei dem die eukaryotischen Zellen im Transportkanal hydrodynamisch fokussiert werden, um sie im Wesentlichen hintereinander in einer hydrodynamischen Fokussierungsebene (P) oder im Wesentlichen entlang einer hydrodynamischen Fokussierungsachse auszurichten, und wobei der Abstand (D₃) zwischen dem Auslass (104b) des ersten Durchgangs (104) im Transportkanal (100) und der hydrodynamischen Fokussierungsebene (P) oder der hydrodynamischen Fokussierungsachse der eukaryotischen Zellen (SP) im Transportkanal (100) weniger als 1 mm, weiter bevorzugt weniger als 100 µm und noch weiter bevorzugt sogar weniger als 50 µm beträgt, und/oder wobei der Abstand (D₁), zwischen dem Ausgang (102b) des Durchgangskanals (102) die Fluoreszenzanregung im Transportkanal (100) und der hydrodynamischen Fokussierungsebene (P) oder der hydrodynamischen Fokussierungsachse von eukaryotischen Zellen (SP) im Transportkanal (100), weniger als 1 mm, bevorzugter weniger als 100 µm und bevorzugter sogar weniger als 50 µm beträgt, und/oder wobei der Abstand zwischen dem Auslass (103b) des Durchgangskanals (103) zur Fluoreszenzdetektion im Transportkanal (100) und der hydrodynamischen Fokussierungsebene (P) oder der hydrodynamischen Fokussierungsachse von eukaryotischen Zellen (SP) im Transportkanal (100) weniger als 1 mm, bevorzugter weniger als 100 µm und bevorzugter sogar weniger als 50 µm beträgt.

16. Verfahren nach einem der Ansprüche 14 bis 15, wobei die eukaryontischen Zellen Spermien (SP) sind, insbesondere tierische Spermien, die unterschiedliche Chromosomen aufweisen können.

## Claims

1. Device for selecting eukaryotic cells (SP), which device comprises a transport channel (100) in which a solution containing said eukaryotic cells (SP) can circulate, a first through-passage (104) leading into said transport channel (100), an electromagnetic radiation source (60) which is coupled to a first end of a first optical fiber (61), the other emission end (61b) of the first optical fiber being inserted into said first through-passage (104) without projecting into the transport channel (100), the transport channel (100) being a microfluidic channel of which at least one cross-sectional dimension is less than 1 mm, and more particularly less than 100 µm, and said device further comprising electronic control means (7) for automatically controlling said electromagnetic radiation source (60) so as to selectively damage the eukaryotic cells (SP) circulating in the transport channel (100) by means of damaging electromagnetic radiation (R) emitted by the electromagnetic radiation source (60).

2. Device according to claim 1, comprising optical focusing means which are attached to or integrated into the emission end (61b) of the first optical fiber and which make it possible to focus, in said transport channel (100), the electromagnetic radiation (R) emitted at the outlet of the first optical fiber (61), or optical focusing means formed by the emission end (61b) of the first optical fiber (61) which is profiled so as to focus, in said transport channel (100), the electromagnetic radiation (R) emitted at the outlet of the optical fiber (61).

3. Device according to claim 2, wherein the emission end (61b) of the first optical fiber (61) is conical, or wherein the emission end (61b) of the first optical fiber (61) is wedge-shaped.

4. Device according to any of the preceding claims, wherein the emission end (61b) of the first optical fiber is flush with the transport channel (100) without projecting into the transport channel.

5. Device according to any of the preceding claims, wherein the transport channel (100) has a rectangular cross section, and preferably wherein the width (E) of the rectangular transport channel (100) is less than 1 mm, and preferably less than 100 µm.

6. Device according to any of the preceding claims, comprising a second electromagnetic radiation source (40) capable of emitting electromagnetic excitation radiation which makes it possible to excite the fluorescent emission of the eukaryotic cells (SP) circulating in said transport channel (100), and at least one photodetector (50) for detecting the fluorescence emitted by said eukaryotic cells (SP), and wherein the electronic control means (7) are capable of processing an electrical detection signal (50a) output by said photodetector (50) and controlling the first electromagnetic radiation source (60) in order to selectively damage eukaryotic cells (SP) on the basis of this detection signal (50a).

7. Device according to claim 6, comprising a through-passage (102) for fluorescence excitation, which passage leads into said transport channel (100) upstream of the first through-passage (104), and wherein the second electromagnetic radiation source (40) is coupled to a first end of a fluorescence excitation optical fiber (41), the other emission end (41b) of said fluorescence excitation optical fiber (41) being inserted into said through-passage (102) for the fluorescence excitation without projecting into the transport channel (100).

8. Device according to either claim 6 or claim 7, comprising a through-passage (103) for fluorescence detection, which passage leads into said transport channel (100) upstream of the first through-passage (104), and wherein one end (41b) of a fluorescence detection optical fiber (51) is inserted into said through-passage (103) without projecting into the transport channel (100), and the other emission end (51b) of said fluorescence detection optical fiber (51) being associated with the photodetector (50).

9. Device according to claim 8, wherein the distance (E) between the outlet (102b) into the transport channel (100) of the through-passage (102) for fluorescence excitation and the opposite wall of the transport channel (100) is less than 1 mm, more preferably less than 100 µm, and/or wherein the distance (E) between the outlet (103b) into the transport channel (100) of the through-passage (103) for fluorescence detection and the opposite wall of the transport channel (100) is less than 1 mm, more preferably less than 100 µm.

10. Device according to any of the preceding claims, wherein the distance (E) between the outlet (104b) into the transport channel (100) of the first through-passage (104) and the opposite wall of the transport channel (100) is less than 1 mm, more preferably less than 100 µm, and even more preferably less than 50 µm.

11. Device according to any of the preceding claims, comprising means (2) for injecting a solution (S) containing eukaryotic cells (SP), and preferably eukaryotic cells (SP) labeled with at least one fluorochrome, into said transport channel (100), and comprising hydrodynamic focusing means (3; 101) for injecting a liquid (L) into the transport channel (100) so as to drive the eukaryotic cells (SP) in the transport channel (100), positioning them substantially in a hydrodynamic focusing plane (P) or substantially along a hydrodynamic focusing axis and spacing them apart one behind the other.

12. Device according to claim 11 and any of claims 2 to 5, wherein said optical focusing means make it possible to focus the damaging electromagnetic radiation (R) substantially in said hydrodynamic focusing plane (P) of the eukaryotic cells (SP) or substantially on the hydrodynamic focusing axis of the eukaryotic cells (SP).

13. Use of the device according to any of the preceding claims for automatically selecting eukaryotic cells, more particularly sperm cells, and even more particularly animal sperm cells, in vitro.

14. Method for in vitro selection of eukaryotic cells (SP) that can be of different types, making it possible to categorize them into at least two distinct categories, by means of a selection device according to any of claims 1 to 12, during which method a solution (S) containing the eukaryotic cells (SP) to be sorted is injected into the transport channel (100) of the sorting device, said eukaryotic cells (SP) are caused to circulate in the transport channel (100) one behind the other, the type of each eukaryotic cell circulating in the transport channel (100) is automatically detected, and the eukaryotic cells (SP) which have been detected as being of the same predefined type are selectively irradiated with the damaging electromagnetic radiation (R) so as to damage them sufficiently in order to render them non-viable, the other eukaryotic cells not being damaged by the damaging electromagnetic radiation (R).

15. Method according to claim 14, during which the eukaryotic cells are hydrodynamically focused in the transport channel so as to align them one behind the other substantially in a hydrodynamic focusing plane (P) or substantially along a hydrodynamic focusing axis, and wherein the distance (D₃) between the outlet (104b) into the transport channel (100) of the first through-passage (104) and the hydrodynamic focusing plane (P) or the hydrodynamic focusing axis of the eukaryotic cells (SP) in the transport channel (100) is less than 1 mm, more preferably less than 100 µm, and even more preferably less than 50 µm, and/or wherein the distance (D₁) between the outlet (102b) into the transport channel (100) of the through-passage (102) for fluorescence excitation and said hydrodynamic focusing plane (P) or said hydrodynamic focusing axis of the eukaryotic cells (SP) in the transport channel (100) is less than 1 mm, more preferably less than 100 µm, and even more preferably less than 50 µm, and/or wherein the distance between the outlet (103b) into the transport channel (100) of the through-passage (103) for fluorescence detection and the hydrodynamic focusing plane (P) or the hydrodynamic focusing axis of the eukaryotic cells (SP) in the transport channel (100) is less than 1 mm, more preferably less than 100 µm, and even more preferably less than 50 µm.

16. Method according to any of claims 14 to 15, wherein the eukaryotic cells are sperm cells (SP), specifically animal sperm cells, which can have different chromosomes.
